# EUROPEAN PATENT APPLICATION

(11) **EP 4 846 959 A2**
(43) Date of publication of application: **02.12.2026**
(21) Application number: 26181773.8
(22) Date of filing: 28.05.2026
(51) Int. Cl.: A61B 17/72, A61B 17/17, A61B 17/80, A61B 17/00, A61B 17/16

(54) **HUMERAL PLATE AND NAIL SYSTEMS**

(30) Priority: 30.05.2025 US 202519223831
(71) Applicant: Globus Medical, Inc., Audubon, PA 19403 (US)
(72) Inventor: INGLIS, Brendan, PHOENIXVILLE, 19460 (US); SOLASKI, Banjamin, NEW YORK, 10002 (US); BAYYARI, Malak, PHOENIXVILLE, 19460 (US); KIRK, Amanda, NEWTOWN SQUARE, 19073 (US)
(74) Representative: Morabito, Sara

(57) **Abstract**

A humeral plate and nail system is disclosed for orthopedic surgical procedures on the humerus, particularly for complex proximal humeral fractures. The system includes an intramedullary humeral nail and a humeral plate positioned laterally adjacent to the bicipital groove. The plate is equipped with removably connectable extension flanges that can be intra-operatively bent to conform to the anterior or posterior surfaces of the humerus, improving stability. The system may also include radiolucent components, such as a radiolucent drill, radiolucent drill guide, and radiolucent wire guide

## Description

### FIELD

This disclosure relates to orthopedic surgical systems in general, and to systems and related devices and methods for performing orthopedic surgical procedures on the humerus particularly.

### BACKGROUND

Humeral nails and proximal humerus/humeral plates are currently used by orthopedic surgeons as internal fixation devices to address associated facture patterns. Such patterns may primarily include fixation of proximal humerus fractures, but may likewise address other indications, such as buttressing of comminuted/multi-fragmentary fractures, extra-articular proximal humerus fractures (that is, below surgical neck/humeral shaft fractures), fractures in osteopenic bones, nonunions and malunions, and oncology deformations.

In such applications, a humeral nail is generally placed in the intramedullary canal of the humerus through an entry point generally in the top of the humeral head.

While humeral plates may be contemplated or considered for humeral procedures, the anatomy and physiology of the humerus and surrounding bones needs to factored into the humeral plate and related devices and methods contemplated by the system. It may likewise be desirable for the nails, plates and related components of humeral surgical systems to be configured so as to accommodate a variety of fracture patterns, or to be otherwise easily and readily adaptable by the surgeon or other medical practitioner to address the variables associated with such fracture patterns either *in situ* or through the associated surgical procedure plan.

In addition to the foregoing, certain surgical instruments and devices are often used under fluoroscopy during surgical procedures on the humerus, and certain modifications to the features of such instruments may address the challenges of performing procedures under such conditions.

### SUMMARY

In one implementation, a humeral plate and nail system is designed for orthopedic surgical procedures involving the patient's humerus. The system comprises an intramedullary humeral nail and a specially contoured humeral plate. The plate is configured to be positioned laterally adjacent to the bicipital groove at the proximal portion of the humerus. This configuration allows the system to provide optimal anatomical alignment and structural support, facilitating treatment of complex humeral fractures, particularly those involving the proximal humerus.

In another potential implementation, the humeral plate is designed with a plurality of polyaxial holes. These holes are strategically oriented to allow the insertion of surgical fasteners, such as screws, along trajectories that intersect both the intramedullary nail and the surrounding humeral bone. Such trajectories enhance the stability of the fixation construct by creating cross-locking points between the plate, the nail, and the bone. This arrangement significantly improves the overall angular stability of the system, allowing it to handle multi-fragmentary fractures more effectively.

In still another potential implementation, the humeral plate further incorporates removably connectable extension flanges. These flanges are designed to be intra-operatively adjustable and bendable by the surgeon, enabling them to contour the flanges as needed to oppose the anterior or posterior surfaces of the humerus. This additional support further enhances fixation strength, especially in cases where additional stabilization is required due to complex fracture patterns. The modular nature of these flanges allows the system to be customized during surgery based on the patient's specific anatomical requirements.

In still further implementations, the intramedullary humeral nail includes a cylindrical body with multiple strategically positioned bores that are configured to receive locking screws. These bores include a calcar bore designed to provide medial column support, which is critical for maintaining structural integrity and preventing varus collapse. Additionally, proximal bores are placed to enable rafting of the humeral head, thereby supporting articular fragments. The system is further equipped with a set screw mechanism that allows the surgeon to apply active compression. This feature promotes better fracture reduction, stabilization, and promotes early healing.

In an additional implementation, the system further comprises a radiolucent drill comprising a steel shaft for strength and a radiolucent quick-connect for secure attachment to a drill guide. The radiolucent material reduces fluoroscopic interference, allowing clear visualization of the drill's position and facilitating accurate targeting of the humeral nail's distal holes.

The invention further includes a radiolucent drill guide (RLDG) that improves alignment and accuracy in drilling. The drill guide incorporates an extension handle for stable maneuverability, a drill chuck for secure drill attachment, and a radio-opaque aiming circle for precise targeting. The reusable RLDG features a variable power transfer mechanism, enabling efficient drilling at adjustable angles. A disposable RLDG variant is also provided, featuring a lightweight radiolucent plastic handle and a reinforced metal insert sleeve for enhanced durability and imaging clarity under fluoroscopy.

Additionally, the system includes a radiolucent wire guide designed to assist in the precise marking of nail holes. The wire guide features a radiolucent handle and housing to minimize imaging obstruction and a set screw for securing the wire. The open-back design enables stable wire placement before drilling, reducing the risk of misalignment. The wire guide is compatible with the radiolucent drill and drill guide, ensuring seamless integration into surgical workflows.

This radiolucent drilling system provides improved accuracy, efficiency, and visualization in orthopedic procedures requiring precise drilling through a humeral nail. The combination of radiolucent materials and strategic design elements minimizes fluoroscopic obstruction while maintaining structural integrity, offering an effective solution for enhanced surgical outcomes.

The foregoing summary and this disclosure will be better understood with reference to the drawings.

### BRIEF DESCRIPTION OF DRAWINGS

A more complete understanding of the present disclosure, and the attendant advantages and features thereof, will be more readily understood by reference to the following detailed description, when considered in conjunction with the accompanying drawings, wherein:
Fig. 1 is an illustrative view showing a humeral plate and nail system for orthopedic surgery according to the present disclosure.
Fig. 2 is a zoomed in view showing the humeral nail fixed with four screws for stabilization according to the present disclosure.
Fig. 3 is an illustrative view showing the anterior-posterior (AP) flanges for enhanced fixation and stability according to the present disclosure.
Fig. 4 is a perspective view showing an exemplary humeral nail designed for internal fixation of the humerus according to the present disclosure.
Fig. 5 is an illustrative view showing a set screw providing compression within the slot of the humeral nail according to the present disclosure.
Fig. 6 is a perspective view showing a radiolucent drill according to the present disclosure.
Fig. 7 is an illustrative view showing a 90-degree drill guide according to the present disclosure.
Fig. 8 is an exploded view showing a disposable radiolucent drill guide (RLDG) according to the present disclosure.
Fig. 9 is an illustrative view showing a radiolucent wire guide, according to the present disclosure.

### DETAILED DESCRIPTION

In one implementation, a humeral plate and nail system 100 is disclosed with reference to Figs.

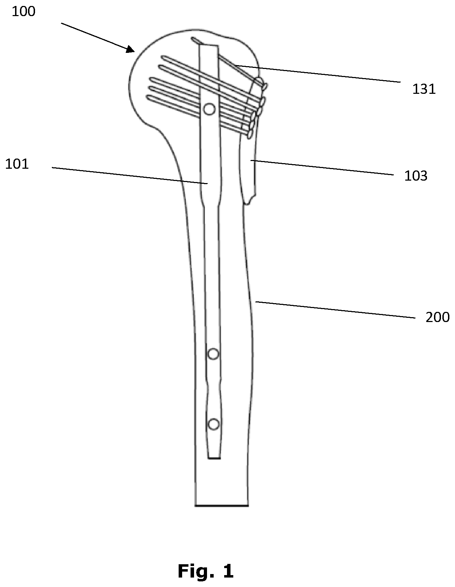

Referring to Fig. 1, it depicts the humeral plate and nail system 100 designed for orthopedic surgical fixation of the humerus 200, specifically targeting proximal humeral fractures. The humeral plate and nail system 100 comprises a humeral nail 101 and a humeral plate 103. The humeral nail 101 is shown inserted intramedullary into the humerus 200. Humeral plate 103 is positioned laterally adjacent to the bicipital groove of the proximal humerus. The humeral plate 103 is designed to receive surgical screws 131 at various angles. One set of screws intersects the humeral nail 101 for enhanced nail-plate integration. Another set bypasses the nail to anchor directly into the humerus, optimizing bone fixation. The anatomical contour of the humeral plate 103 allows it to conform to the proximal humerus surface.

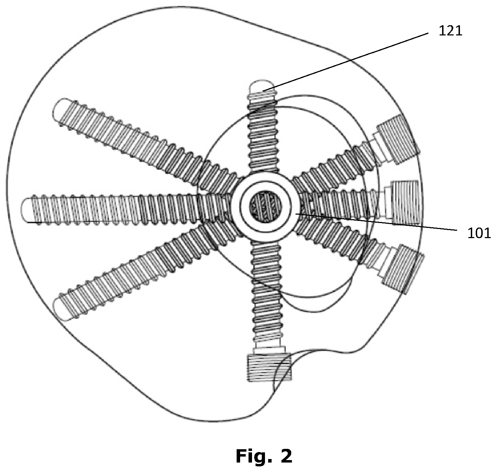

Fig. 2 provides a focused zoomed in view of the humeral nail 101 positioned within the humerus 200 and stabilized using four locking screws 121. Three of these screws are positioned through the greater tuberosity area, aiding in rafting the humeral head. One screw is placed targeting the lesser tuberosity at a 90-degree angle relative to the longitudinal axis, improving fixation stability. The arrangement shows angular offsetting of 25 degrees between the three greater tuberosity screws, enhancing the rafting effect. This view emphasizes how the locking screws 121 penetrate through specific bores in the nail to secure the proximal humerus effectively.

The humeral nail further includes a dedicated calcar bore. This bore is positioned at an approximate 45-degree angle in a distal-to-proximal orientation. The calcar bore provides enhanced support to the medial column of the humerus, thereby improving structural stability in osteoporotic or comminuted fractures. The humeral nail further includes distal bores. The distal bore may be positioned with two bores in line and one bore with a 25-degree radial offset relative to the other bores. This configuration enhances rotational stability of the implant within the medullary canal, ensuring better fixation and resistance to torsional forces during postoperative rehabilitation.

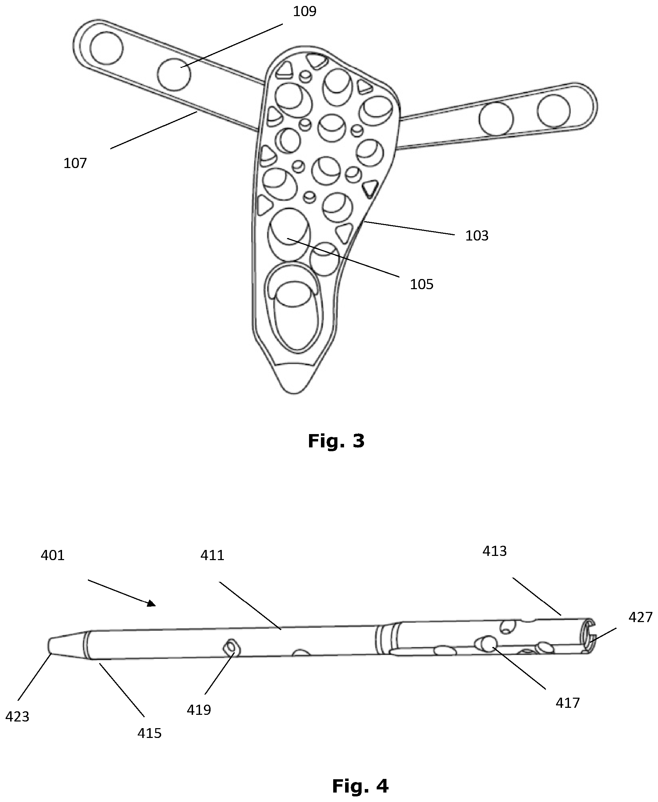

Fig. 3 illustrates the anterior-posterior (AP) extension flanges 107 designed to enhance the fixation and stability of the humeral plate 103. The extension flanges 107 are removably connected to the main body of the plate 103 and extend radially outward. The design allows intra-operative bending of each flange to adapt to the anterior or posterior surfaces of the humerus 200, providing patient-specific fitment. Extension flange holes 109 are depicted in the flanges to receive surgical screws 131 that anchor the flange into the humerus. The AP flanges provide multidirectional stabilization, increasing the structural integrity of the implant and improving fixation in complex fractures.

This figure specifically highlights the versatility of the AP flanges, showing how one flange is positioned against the anterior surface while the other is adapted to the posterior surface. The humeral plate 103 includes a plurality of polyaxial holes 105 designed to receive corresponding fasteners 131 at variable angles. Humeral plate 103 may also have monoaxial holes. These polyaxial holes 105 enable both direct engagement with the humerus 200 and intersection through the humeral nail 101 to enhance fixation stability.

Fig. 4 presents a detailed perspective view of a humeral nail 401 designed for internal fixation. The humeral nail 401 comprises a cylindrical body 411 with its proximal end 413 and distal end 415. The humeral nail features a tapered distal tip 423, which may be fitted to the distal aspect of the medullary canal and may facilitate easier insertion into the medullary canal while minimizing stress during implantation. Additionally, the nail maintains a straight longitudinal axis, ensuring proper alignment within the humeral shaft and reducing the risk of mispositioning. The figure showcases the first group of bores 417 positioned near the proximal end 413 for proximal humeral fixation and rafting of the humeral head. Four locking screws 421 are inserted through these bores 417, providing four rows of fixation. Additionally, the second group of bores 419 is illustrated toward the distal end 415, designed to receive locking screws 421 that prevent rotation and enhance axial stability.

A longitudinally extending slot 425 may be at the proximal end 413, which includes a threaded bore 427 for accommodating a set screw 428. This design allows for active compression mechanisms. The humeral nail is fabricated from a biocompatible anodized titanium alloy, which offers high mechanical strength and corrosion resistance. The anodization process enhances the surface properties of the nail and reducing wear over time.

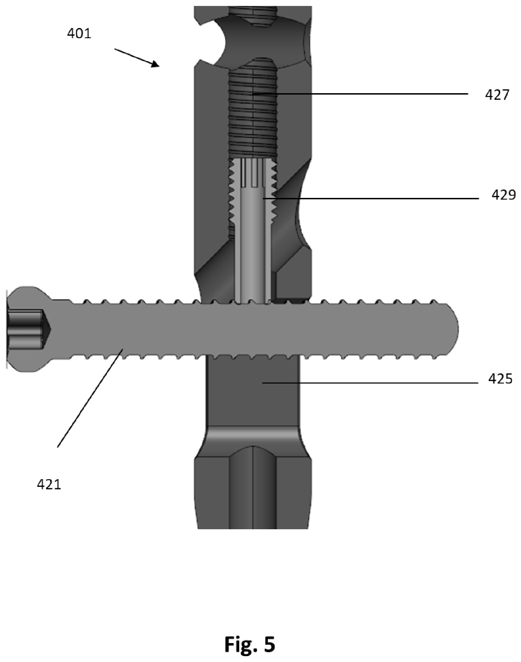

Fig. 5 focuses on the set screw 429 mechanism incorporated into the humeral nail 401. The longitudinal slot 425 is shown with the threaded bore 427 running within it. The set screw 429 is threaded through the bore and shown advancing in the distal direction. The set screw 429 is positioned to engage one of the locking screws, such as locking screw 421. This interaction demonstrates how the set screw 429 applies compression onto the locking screw 421 to enhance fixation.

The figure also suggests the possible inclusion of a spring mechanism or nitinol spring (not shown) to provide active compression of the fastener, improving the stability of the construct. This configuration allows fine-tuned compression of the screws post-implantation, optimizing fracture healing and fixation strength.

The humeral nail 101 is equipped with a threaded bore 127 designed to receive a set screw 129. This set screw 129 mechanism enables controlled compression of the fastener against the bone, thereby improving construct stability and to reduce the risk of non or mal unions.at the fracture site. The set screw 129 also provides additional resistance against screw backout during postoperative movement.

The system includes a variety of surgical screws for different fixation needs. These screws may be headless for improved countersinking, undercut for optimal engagement, or feature blunt tips to prevent soft tissue irritation. This selection allows surgeons to choose the most suitable screw type based on bone quality and fracture configuration.

The system 100 is available as a set of humeral nails 101 having varying lengths and diameters to accommodate different patient anatomies and fracture types. The set may include short nails for proximal and articular fractures and long nails suitable for mid-shaft humeral fractures.

To accommodate different fracture patterns and patient anatomies, the humeral nail 101 is available in multiple lengths. The varying lengths allow the nail to be used effectively in treating proximal, midshaft, and segmental humeral fractures. This adaptability provides surgeons with options for optimizing patient outcomes based on the severity and location of the fracture.

During surgical fixation, the humeral plate 103 is positioned laterally adjacent to the bicipital groove. Locking screws 121 are inserted through the bores 117 in the humeral nail 101 at angles that provide maximum fixation of the humeral head and tuberosities. Simultaneously, fasteners 131 may be inserted through the polyaxial holes 105 of the plate at both nominal and variable angles, allowing some to intersect the nail and others to engage the humerus directly.

The surgical technique involves first inserting the humeral nail 101 into the medullary canal, humeral nail is locked distally, compression is applied, and the humeral plate 103 is aligned and positioned. Once positioned, polyaxial screws are introduced through the plate 103 and into the nail's pre-configured bores. The set screw 129 is then engaged to provide additional compression and stability. Finally, the extension flanges are intra-operatively bent to contour the plate to the patient's humerus, optimizing anatomical fit and load distribution.

The system 100 enables multi-planar fixation, improved load distribution, and enhanced stability for proximal humeral fractures, especially complex fracture patterns requiring dual fixation. The modular and intra-operatively adjustable design allows surgeons to customize fixation based on the fracture characteristics and patient anatomy.

In the best mode contemplated, the humeral plate and nail system 100 is fabricated from a titanium alloy to provide a balance of high strength, corrosion resistance, and biocompatibility. Titanium alloy also minimizes imaging artifacts during post-operative radiographic monitoring.

The humeral nail 101 is preferably cannulated to allow guided insertion over a guide wire, improving accuracy during intramedullary placement. The cannulation further permits intraoperative irrigation if needed. The proximal end 113 of the nail 101 is designed to accommodate the set screw 129 for controlled compression. Ideally, the set screw 129 is made of stainless steel for higher thread strength, ensuring reliable locking and compression when engaged into the threaded bore 127.

For optimal versatility, the polyaxial holes 105 in the humeral plate 103 are configured to accept variable-angle locking screws, allowing up to 20 degrees of angular adjustment. This enhances the surgeon's ability to target specific fracture fragments and avoid critical anatomical structures such as the axillary nerve.

During surgical implantation, the best practice involves initially securing the humeral nail 101 intramedullary, followed by placement of the humeral plate 103 along the lateral aspect of the humerus 200. The extension flange 107 is pre-contoured or adjusted intraoperatively based on the patient's anatomy, especially for fractures extending distally. Surgical screws 131 inserted through the extension flange holes 109 add fixation strength in comminuted fractures.

The set screw 129 is finally tightened to apply controlled compression across the fracture site. This compression mechanism is particularly advantageous in osteoporotic bone, where fracture gaps often persist after screw fixation. The locking between the set screw 129 and the slot 125 prevents loss of compression over time.

The overall system is designed to allow staged fixation, starting with the nail, followed by plate placement, then screw fixation, and finally compression. This sequence provides flexibility to address different fracture patterns while ensuring stable fixation and promoting optimal healing conditions.

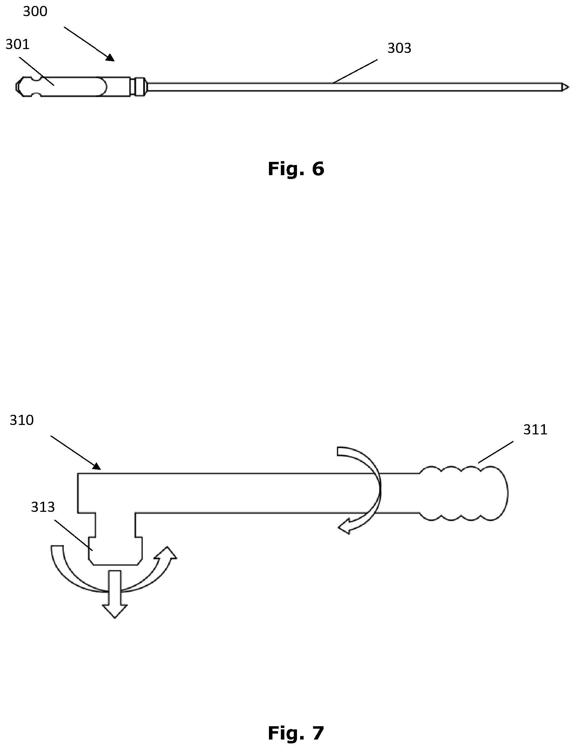

Fig. 6 illustrates a radiolucent drill 300 comprising a steel shaft 303 and a radiolucent quick-connect (connector) 301 made from a durable plastic material. The steel shaft 303 provides the strength and rigidity required for drilling through bone and implant materials, ensuring reliable surgical performance. The radiolucent quick-connect 301 securely locks into the radiolucent drill guide, facilitating precise alignment. Its radiolucent material minimizes interference in fluoroscopic imaging, enabling surgeons to clearly visualize the drill's position. This design ensures smooth operation and accurate targeting of the humeral nail's distal holes.

The radiolucent drill 300 may be used as part of a drilling subsystem, which includes a suitable drill driver for advancing the drill 300 into the humerus and a radiolucent drill guide 310 (Fig. 7) to enhance the accuracy of distal humerus drilling. This drilling subsystem enables the "perfect circle" technique by optimizing visibility under fluoroscopy. The radiolucent connector 301 at the proximal end ensures precise alignment with the humeral nail, reducing the risk of misalignment. Since only the minor diameter of the drill remains visible under fluoroscopy, surgical accuracy is significantly improved. This combination of strength and enhanced visualization provides surgeons with a reliable tool for precise hole placement with minimal fluoroscopic obstruction.

Fig. 7 discloses one possible implementation of radiolucent drill guide (RLDG) 310 suitable for precision in distal humerus drilling using the perfect circle technique. It features an extension handle 311 and a drill chuck 313, ensuring optimal control and ease of use. The extension handle 311 provides a stable grip, allowing surgeons to maneuver the guide while maintaining visibility of the drilling site. The drill chuck 313 securely holds the radiolucent drill 300, ensuring stable connectivity for precise hole placement. Additionally, the guide 310 incorporates a radio-opaque aiming circle within the chuck portion, appearing under fluoroscopy to provide an additional visual reference for accurate nail hole targeting.

The RLDG 310 features a radiolucent housing, making the drill guide transparent under fluoroscopy so that only the drill point above the nail hole remains visible. This allows surgeons to maintain a clear line of sight while drilling. The guide also includes a right-angle or variable power transfer mechanism, enabling power transmission at a fixed or adjustable angle. This facilitates efficient drilling while ensuring alignment with the nail holes.

In one possible implementation, the RLDG 310 is reusable. and designed for disassembly, and/or suitable for hospital sterilization. In certain versions, RLDG 310 may be compatible with a variety of drill accessories, and may make use of a quick-connect system for seamless attachment to one or more manufacturers' drill accessories, ensuring versatility in surgical settings.

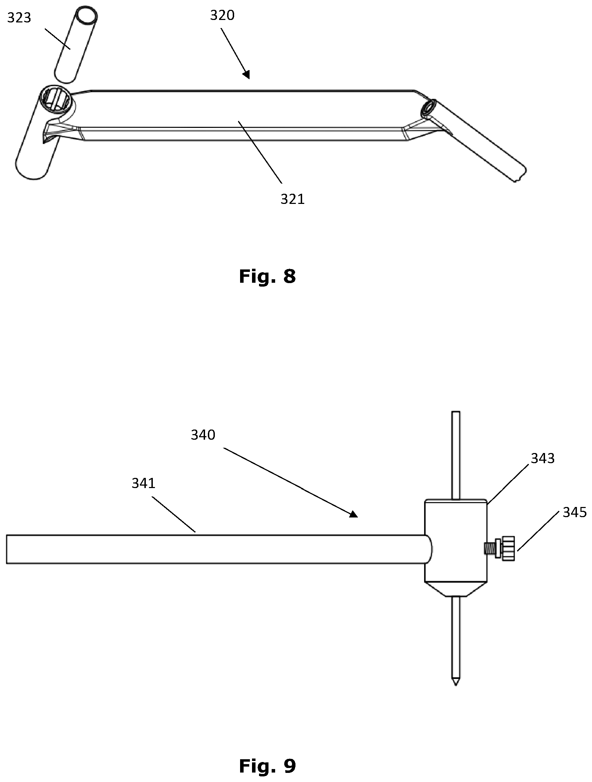

Fig. 8 illustrates a disposable radiolucent drill guide (RLDG) 320, designed as a cost-effective solution for the perfect circle technique. It consists of a plastic handle 321 and a metal insert sleeve 323, ensuring both durability and visibility during the procedure. The lightweight plastic handle 321 may be manufactured using additive techniques, making it radiolucent under fluoroscopy and allowing for accurate drill alignment without significant imaging interference. The metal insert sleeve 323, made of radio-opaque steel or another hard metal, reinforces structural integrity and prevents wear on portions of the guide 320 in contact with moving components during drilling.

In the illustrated implementation, the disposable RLDG 320 features a large handle for adequate soft tissue clearance, providing surgeons with a firm grip while stabilizing the guide during perfect circle drilling. It is compatible with the radiolucent drill 300, ensuring precise hole placement while minimizing fluoroscopic obstruction. The disposable design offers a practical, sterile, and ready-to-use instrument for single-use applications. With its combination of a radiolucent plastic housing and a reinforced metal insert sleeve 323, the disposable RLDG 320 ensures accuracy, safety, and efficiency in orthopedic procedures requiring precise drilling through a humeral nail.

Fig. 9 discloses a radiolucent wire guide 340 that assists in accurately targeting nail holes, including those associated with the perfect circle method. Wire guide 340 includes a radiolucent handle 341, a housing 343, and a set screw 345 for wire retention. The radiolucent handle 341 and housing 343 may be made from radiolucent material under fluoroscopy, again, to ensure minimal obstruction while aligning the wire guide 340 with the humeral nail. The set screw 345 secures the wire in place, allowing precise marking of the start point for drilling and reducing the risk of misalignment.

The radiolucent wire guide 340 features an open-back design with a through-hole at the proximal end, allowing the wire to be impacted or drilled into place to create a stable starting hole for distal nail hole drilling. This ensures proper wire placement before drilling, enhancing procedural accuracy and efficiency. Additionally, the wire guide 340 is compatible with the radiolucent drill 300 and drill guide 310, ensuring seamless integration into the surgical workflow. Its lightweight yet durable construction facilitates ease of use while maintaining precision for orthopedic procedures involving the perfect circle technique.

The present invention provides an efficient solution for perfect circle targeting in distal humeral nailing. By maximizing drill point and nail visibility under fluoroscopy, the radiolucent drill guide (RLDG) ensures precise alignment and consistent drill trajectory. The 90-degree or variable angle power transfer allows surgeons to operate outside the fluoroscopy viewing window, overcoming spatial limitations of miniature C-Arm fluoroscopes. This prevents interference with imaging while keeping the drill bit and patient anatomy under fluoroscopic guidance.

Additionally, the RLDG features an aiming circle, offering surgeons a crucial visual reference for accurate targeting of distal nail holes and minimizing misalignment risks. The disposable radiolucent drill guide introduces an innovative 3D-printed targeting solution, significantly improving visualization compared to conventional drill guides in humeral nailing. These advancements collectively enhance surgical accuracy, efficiency, and safety, setting a new standard for orthopedic procedures utilizing the perfect circle technique.

It will be further understood that still further changes and variations in the details, materials, and arrangements of the components, parts, and features which have been described and illustrated may be made. All such modifications are deemed to be within the scope of this disclosure, as defined by the appended claims.

The invention could be inter alia defined by the following examples:
1. A humeral plate and nail system for orthopedic surgery procedures on a patient's humerus, the system comprising: a humeral nail configured for intramedullary insertion; and a humeral plate having a predetermined contour and size to be positionable laterally adjacent to the bicipital groove at the proximal end of the humerus; wherein the humeral plate includes a plurality of polyaxial holes defined therein, the holes located and oriented to receive corresponding ones of the fasteners at corresponding trajectories, the trajectories including at least a first trajectory intersecting the nail and a second trajectory into the humerus and outside the nail.
2. The system of Example 1, wherein the contour of the humeral plate terminates in a first circumference, and wherein the humeral plate includes at least one extension flange extending in a corresponding plane and radially outwardly from the first circumference to define a second circumference, the extension flange configured to be bendable intra-operatively from the corresponding plane and thereby orientable to oppose one of the humeral surfaces anterior or posterior to the humeral plate, the extension flange having at least one hole defined therein for receiving a corresponding one of the fasteners to engage the humerus at the corresponding location of the hole.
3. The system of Example 2, comprising two of the extension flanges, the extension flanges being removably connectable to the humeral plate, one of the flanges being bendable intra-operatively to oppose the posterior of the humerus and the other of the flanges being bendable intra-operatively to oppose the anterior of the humerus, when the humeral plate has been positioned laterally adjacent to the bicipital groove of the proximal humerus.
4. The system of Example 1, wherein the humeral nail comprises a cylindrical body having a proximal end and a distal end and extending a predetermined distance between the ends along a longitudinal axis of the body; wherein the humeral nail comprises a plurality of bores defined at predetermined locations along the longitudinal axis, extending through the cylindrical body, and intersecting the longitudinal axis at predetermined angles; and wherein the humeral plate and nail system further comprises a plurality of locking screws configured to be received through corresponding ones of the bores to affix the nail relative thereto.
5. The system of Example 4, wherein the plurality of bores comprises a first group of bores disposed at the proximal end of the humeral nail at four discrete, predetermined, longitudinally spaced locations to define four rows of fixation when the corresponding locking screws are received therethrough.
6. The system of Example 5, wherein the first set of bores comprises four bores, one at each of the discrete, predetermined locations, the predetermined locations of three of the four bores corresponding to the greater tuberosity and the predetermined location of one of the four bores corresponding to the lesser tuberosity.
7. The system of Example 6, wherein the predetermined angles of each of the three bores are selected to be offset from an adjacent one of the three bores by a 25-degree angle measured radially relative to the longitudinal axis of the cylindrical body to raft the humeral head when secured thereto; wherein the three bores are disposed so that one of the three bores defines a primary, greater tuberosity bore; and wherein the one bore corresponding to the lesser tuberosity is disposed at a 90-degree angle measured radially relative to the longitudinal axis.
8. The system of Example 4, wherein the plurality of bores comprises a calcar bore having a corresponding, predetermined location in operative proximity to the medial calcar region of the proximal humerus when the nail is inserted therein, the predetermined angle of the calcar bore having a distal-to-proximal angular orientation of 45 degrees.
9. The system of Example 4, wherein the plurality of bores comprises a second group of bores comprising at least two bores at predetermined locations on the distal end of the nail and wherein respective, predetermined angles of the bores of the second set are selected to be offset from each other by a 25-degree angle measured radially relative to the longitudinal axis of the cylindrical body to inhibit unintended rotation of the nail about the longitudinal axis after the surgical procedure.
10. The system of Example 4, wherein the cylindrical body tapers to form a distal tip.
11. The system of Example 4, wherein the longitudinal axis of the cylindrical body is straight to facilitate insertion into a medial portion of the humerus.
12. The system of Example 4, wherein the nail consists essentially of an anodized titanium alloy.
13. The system of Example 4, wherein the system comprises a set of humeral nails having predetermined lengths and diameters.
14. The system of Example 13, wherein the set comprises at least one short nail, a predetermined length and diameter of which are adapted for use with at least one of articular and proximal fractures.
15. The system of Example 14, wherein the humeral nail set comprises at least one long nail, a predetermined length and diameter of which are adapted for use with mid-shaft humeral fractures.
16. The system of Example 4, wherein the proximal end of the cylindrical body has a longitudinally extending slot defined therein, the slot having a threaded bore defined therein terminating in a proximal opening at the proximal end of the slot, the slot communicating with at least one of the bores of the first group of bores located at the proximal end of the nail; wherein the system includes at least one set screw selectively advanceable through the threaded bore in the distal direction and sized to be engageable with one of the fasteners when received in the at least one bore of the first group of bores.
17. The system of Example 16, wherein the humeral nail includes means to actively compress the fastener when engaged by the set screw, and wherein the active compression means comprises at least one of a spring mechanism disposed in the set screw and a nitinol spring operatively connected to the slot and the set screw.
18. The system of Example 1, wherein the fasteners include surgical screws configured to have at least one of the following structural elements: headless proximal ends, headed proximal ends, blunt tips, and undercut, helical threads.
19. A humeral plate and nail system for orthopedic surgery procedures on a patient's humerus, the system comprising: a humeral nail configured for intramedullary insertion into the proximal end of the humerus; and a humeral plate having a predetermined contour and size to be positionable laterally adjacent to the bicipital groove at the proximal end of the humerus; wherein the humeral nail includes a plurality of polyaxial holes defined therein, the holes located and oriented to receive corresponding ones of the fasteners at corresponding trajectories, the trajectories including at least a first trajectory intersecting the nail and a second trajectory into the humerus and outside the nail; wherein the contour of the humeral plate terminates in a first circumference, and wherein the humeral plate includes two, removably connectable extension flanges, each of the extension flanges extending in a corresponding plane and radially outwardly from the first circumference, each of the extension flanges configured to be bendable intra-operatively from the corresponding plane and thereby orientable to oppose one of the humeral surfaces anterior or posterior to the humeral plate, the extension flange having at least one hole defined therein for receiving a corresponding one of the fasteners to engage the humerus at the corresponding location of the hole, one of the flanges being bendable intra-operatively to oppose the posterior of the humerus and the other of the flanges being bendable intra-operatively to oppose the anterior of the humerus, when the humeral plate has been positioned laterally adjacent to the bicipital groove of the proximal humerus; wherein the humeral nail comprises a cylindrical body having a proximal end and a distal end and extending a predetermined distance between the ends along a longitudinal axis of the body; wherein the humeral nail comprises a plurality of bores defined at predetermined locations along the longitudinal axis, extending through the cylindrical body, and intersecting the longitudinal axis at predetermined angles; and wherein the humeral plate and nail system further comprises a plurality of locking screws configured to be received through corresponding ones of the bores to affix the nail relative thereto.
20. A method of setting a fracture of the proximal humerus with a humeral nail and humeral plate, the method comprising: positioning the humeral plate for subsequent fixation at a location laterally adjacent to the bicipital groove at the proximal end of the humerus; inserting a plurality of locking screws into the proximal end of the humeral nail through corresponding bores at offset angles to each other to raft the humeral head, one of the locking screws comprising a primary, greater tuberosity, locking screw; inserting a lesser tuberosity locking screw into the proximal end of the humeral nail through a corresponding bore at an angle offset by 90 degrees from the primary greater tuberosity locking screw; inserting surgical fasteners through corresponding polyaxial holes of the humeral plate at both nominal and variable angle trajectories relative to the longitudinal axis, at least one of the trajectories passing through a corresponding one of the bores of the humeral nail and at least another one of the trajectories engaging the humerus outside of the humeral nail; and intra-operatively bending a removably connectable extension flange to oppose one of the posterior and anterior surfaces of the humerus.

## Claims

1. A humeral plate and nail system for orthopedic surgery procedures on a patient's humerus, the system comprising:
- a humeral nail configured for intramedullary insertion; and
- a humeral plate having a predetermined contour and size to be positionable laterally adjacent to the bicipital groove at the proximal end of the humerus;
- wherein the humeral plate includes a plurality of polyaxial holes defined therein, the holes located and oriented to receive corresponding ones of the fasteners at corresponding trajectories, the trajectories including at least a first trajectory intersecting the nail and a second trajectory into the humerus and outside the nail.

2. The system of claim 1, wherein the contour of the humeral plate terminates in a first circumference, and wherein the humeral plate includes at least one extension flange extending in a corresponding plane and radially outwardly from the first circumference to define a second circumference, the extension flange configured to be bendable intra-operatively from the corresponding plane and thereby orientable to oppose one of the humeral surfaces anterior or posterior to the humeral plate, the extension flange having at least one hole defined therein for receiving a corresponding one of the fasteners to engage the humerus at the corresponding location of the hole.

3. The system of claim 1 or 2, comprising two of the extension flanges, the extension flanges being removably connectable to the humeral plate, one of the flanges being bendable intra-operatively to oppose the posterior of the humerus and the other of the flanges being bendable intra-operatively to oppose the anterior of the humerus, when the humeral plate has been positioned laterally adjacent to the bicipital groove of the proximal humerus.

4. The system of any one of the preceding claims, wherein the humeral nail comprises a cylindrical body having a proximal end and a distal end and extending a predetermined distance between the ends along a longitudinal axis of the body; wherein the humeral nail comprises a plurality of bores defined at predetermined locations along the longitudinal axis, extending through the cylindrical body, and intersecting the longitudinal axis at predetermined angles; and wherein the humeral plate and nail system further comprises a plurality of locking screws configured to be received through corresponding ones of the bores to affix the nail relative thereto.

5. The system of claim 4, wherein the plurality of bores comprises a first group of bores disposed at the proximal end of the humeral nail at four discrete, predetermined, longitudinally spaced locations to define four rows of fixation when the corresponding locking screws are received therethrough, wherein preferably the first set of bores comprises four bores, one at each of the discrete, predetermined locations, the predetermined locations of three of the four bores corresponding to the greater tuberosity and the predetermined location of one of the four bores corresponding to the lesser tuberosity and wherein preferably the predetermined angles of each of the three bores are selected to be offset from an adjacent one of the three bores by a 25-degree angle measured radially relative to the longitudinal axis of the cylindrical body to raft the humeral head when secured thereto; wherein the three bores are disposed so that one of the three bores defines a primary, greater tuberosity bore; and wherein the one bore corresponding to the lesser tuberosity is disposed at a 90-degree angle measured radially relative to the longitudinal axis.

6. The system of claim 4 or 5, wherein the plurality of bores comprises a calcar bore having a corresponding, predetermined location in operative proximity to the medial calcar region of the proximal humerus when the nail is inserted therein, the predetermined angle of the calcar bore having a distal-to-proximal angular orientation of 45 degrees and wherein preferably the plurality of bores comprises a second group of bores comprising at least two bores at predetermined locations on the distal end of the nail and wherein respective, predetermined angles of the bores of the second set are selected to be offset from each other by a 25-degree angle measured radially relative to the longitudinal axis of the cylindrical body to inhibit unintended rotation of the nail about the longitudinal axis after the surgical procedure.

7. The system of any one of claims 4 -6, wherein the cylindrical body tapers to form a distal tip and wherein preferably the longitudinal axis of the cylindrical body is straight to facilitate insertion into a medial portion of the humerus.

8. The system of any one of claims 4 - 7, wherein the nail consists essentially of an anodized titanium alloy.

9. The system of any one of claims 4 -8, wherein the system comprises a set of humeral nails having predetermined lengths and diameters.

10. The system of claim 9, wherein the set comprises at least one short nail, a predetermined length and diameter of which are adapted for use with at least one of articular and proximal fractures.

11. The system of claim 10, wherein the humeral nail set comprises at least one long nail, a predetermined length and diameter of which are adapted for use with mid-shaft humeral fractures.

12. The system of any one of claims 4-11, wherein the proximal end of the cylindrical body has a longitudinally extending slot defined therein, the slot having a threaded bore defined therein terminating in a proximal opening at the proximal end of the slot, the slot communicating with at least one of the bores of the first group of bores located at the proximal end of the nail; wherein the system includes at least one set screw selectively advanceable through the threaded bore in the distal direction and sized to be engageable with one of the fasteners when received in the at least one bore of the first group of bores.

13. The system of claim 12, wherein the humeral nail includes means to actively compress the fastener when engaged by the set screw, and wherein the active compression means comprises at least one of a spring mechanism disposed in the set screw and a nitinol spring operatively connected to the slot and the set screw.

14. The system of any one of the preceding claims, wherein the fasteners include surgical screws configured to have at least one of the following structural elements: headless proximal ends, headed proximal ends, blunt tips, and undercut, helical threads.

15. A humeral plate and nail system for orthopedic surgery procedures on a patient's humerus, the system comprising:
- a humeral nail configured for intramedullary insertion into the proximal end of the humerus; and
- a humeral plate having a predetermined contour and size to be positionable laterally adjacent to the bicipital groove at the proximal end of the humerus;
- wherein the humeral nail includes a plurality of polyaxial holes defined therein, the holes located and oriented to receive corresponding ones of the fasteners at corresponding trajectories, the trajectories including at least a first trajectory intersecting the nail and a second trajectory into the humerus and outside the nail;
- wherein the contour of the humeral plate terminates in a first circumference, and wherein the humeral plate includes two, removably connectable extension flanges, each of the extension flanges extending in a corresponding plane and radially outwardly from the first circumference, each of the extension flanges configured to be bendable intra-operatively from the corresponding plane and thereby orientable to oppose one of the humeral surfaces anterior or posterior to the humeral plate, the extension flange having at least one hole defined therein for receiving a corresponding one of the fasteners to engage the humerus at the corresponding location of the hole, one of the flanges being bendable intra-operatively to oppose the posterior of the humerus and the other of the flanges being bendable intra-operatively to oppose the anterior of the humerus, when the humeral plate has been positioned laterally adjacent to the bicipital groove of the proximal humerus;
- wherein the humeral nail comprises a cylindrical body having a proximal end and a distal end and extending a predetermined distance between the ends along a longitudinal axis of the body;
- wherein the humeral nail comprises a plurality of bores defined at predetermined locations along the longitudinal axis, extending through the cylindrical body, and intersecting the longitudinal axis at predetermined angles; and
- wherein the humeral plate and nail system further comprises a plurality of locking screws configured to be received through corresponding ones of the bores to affix the nail relative thereto.
